(19) Europäisches Patentamt
European Patent Office
Office européen des brevets

(11) Veröffentlichungsnummer: **0 084 127**
**B1**

(12) # EUROPÄISCHE PATENTSCHRIFT

(45) Veröffentlichungstag der Patentschrift: **02.04.86**

(51) Int. Cl.[4]: **C 07 D 209/28**

(21) Anmeldenummer: **82111622.5**

(22) Anmeldetag: **14.12.82**

(54) **Verfahren zur Herstellung von Acemetacin.**

(30) Priorität: **28.12.81 CH 8306/81**

(43) Veröffentlichungstag der Anmeldung: **27.07.83 Patentblatt 83/30**

(45) Bekanntmachung des Hinweises auf die Patenterteilung: **02.04.86 Patentblatt 86/14**

(84) Benannte Vertragsstaaten: **AT BE DE FR GB IT NL SE**

(56) Entgegenhaltungen:
**DE - A - 2 234 651**
**DE - A - 2 943 125**

**T.W. GREENE "Protective groups in organic synthesis", 1981, John Wiley, New York**

(73) Patentinhaber: **SIEGFRIED AKTIENGESELLSCHAFT, CH-4800 Zofingen (CH)**

(72) Erfinder: **Gnehm, René, Dr., Bornweg 8, CH-4665 Küngoldingen (CH)**
Erfinder: **Weber, Rolf, Dr., Rigiweg 3a, CH-4800 Zofingen (CH)**

(74) Vertreter: **Jaeger, Klaus, Dr. et al, JAEGER & PARTNER Patentanwälte Bergstrasse 48 1/2, D-8035 München-Gauting (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung besteht in einem Verfahren zur Herstellung eines Indolderivates, nämlich der 1-(p-Chlorbenzoyl)-5-methoxy-2-methylindol-3-acetoxyessigsäure. Das Verfahren folgt dem an sich bekannten Weg über die Veresterung von 1-(p-Chlorbenzoyl)-5-methoxy-2-methylindol-3-essigsäure mit einem carboxylseitig eine Schutzgruppe aufweisenden Chloressigsäurederivat unter anschliessender Abspaltung der Schutzgruppe.

Die 1-(p-Chlorbenzoyl)-5-methoxy-2-methylindol-3-acetoxyessigsäure, für welche die WHO die Kurzbezeichnung Acemetacin empfiehlt, ist in DE-OS 2 234 651 beschrieben worden als antiphlogistisch wirkende, wertvolle Weiterentwicklung der seit langem als Antiphlogisticum in therapeutischem Gebrauch befindlichen 1-(p-Chlorbenzoyl)-5-methoxy-2-methylindol-3-essigsäure, für welche sich die Kurzbezeichnung Indometacin eingebürgert hat.

Wie den systematischen Bezeichnungen der beiden genannten Substanzen entnommen werden kann, lässt sich das Acemetacin darstellen als das Derivat des Indometacins, welche resultiert bei Veresterung seiner Carboxylgruppe mit dem α-Hydroxyl der Glykolsäure.

In der Praxis lässt sich die Reaktion aus naheliegenden Gründen nicht in dieser Weise durchführen; die Glykolsäure muss durch eine Carbonsäure ersetzt werden, die am α-Kohlenstoff anstelle der Hydroxylgruppe vorzugsweise ein Chloratom und am Carboxyl eine geeignete Schutzgruppe aufweist. Entscheidende Bedeutung kommt dabei der Wahl der Schutzgruppe zu, weil unter den Bedingungen, die man zur Abspaltung üblicher Schutzgruppen anzuwenden hat, auch die andere Esterbindung des Zielproduktes aufgespalten und gegebenenfalls noch weitere Strukturelemente des delikaten Moleküls beeinflusst werden.

Beim in der erwähnten DE-OS 2 234 651 beschriebenen Herstellungsverfahren hat man das Problem zu lösen versucht, indem man als Schutzgruppe den Benzylrest benützte, weil dieser sich hydrogenolytisch abspalten lässt unter weitgehender Schonung der restlichen Struktur.

In der erwähnten Schrift werden noch andere Zugangswege zum Acemetacin beschrieben, welche nicht über Indometacin führen; allen dort aufgezeigten Verfahren gemeinsam ist aber das Merkmal einer die endständige Carboxylgruppe des letzten Zwischenproduktes schützenden Benzylgruppe, die in einem abschliessenden Verfahrensschritt hydrogenolytisch abgespalten werden muss.

Diese Art der Abspaltung ist jedoch im Vergleich etwa zu einer Hydrolyse mit einem nicht unbeachtlichen technischen Aufwand verknüpft und befriedigt auch vom Resultat her gesehen nicht, weil unter den dabei einzuhaltenden Bedingungen das Chloratom des Chlorbenzoylsubstituenten teilweise ebenfalls abgespalten wird. Die über eine hydrogenolytische Debenzylierung führenden Verfahren befriedigen offenbar so wenig, dass man in der Folge (vgl. DE-OS 2 943 125) ein Verfahren für einen gezielten Aufbau der Indolstruktur entwickelt hat, bei dem man α-(Chlorbenzoyl)-4-methoxyphenylhydrazin-Hydrochlorid durch Umsetzung mit Laevulinoyloxyessigsäure in ein Hydrazon überführt und daraus durch Cyclisierung unter Ammoniakabspaltung zum Zielprodukt mit freier Carboxylgruppe gelangt.

Die vorliegende Erfindung beruht nun darauf, dass eine Schutzgruppe gefunden wurde, welche durch einfache Hydrolyse entfernt werden kann und zwar unter so milden Bedingungen, dass keine andere Bindung innerhalb des Moleküls angegriffen wird und damit die Entstehung von Nebenprodukten praktisch vollständig wegfällt. Diese Schutzgruppe ist der 3,4-Tetrahydropyranylrest, der zwar an sich als Schutzgruppe bekannt ist, nicht aber in der Weise, dass seine überraschende Wirkung im vorliegenden Verfahren zu erwarten gewesen wäre («Protective Groups in organic synthesis», 1981, Seiten 160 und 168).

Das Verfahren der Erfindung ist demzufolge dadurch gekennzeichnet, dass man den 1-(p-Chlorbenzoyl)-5-methoxy-2-methylindol-3-acetoxyessigsäure-tetrahydropyranylester unter Säure-Einwirkung hydrolysiert. Für die Hydrolyse hat sich insbesondere Essigsäure als geeignet erwiesen, wobei zweckmässig bei Temperaturen unter dem Siedepunkt derselben, beispielsweise etwa in der Grössenordnung von 40 bis 60 °C gearbeitet werden soll.

Der als Ausgangsstoff dienende, bisher nicht beschriebene 1-(p-Chlorbenzoyl)-5-methoxy-2-methylindol-3-acetoxyessigsäure-tetrahydropyranylester lässt sich nach für den Fachmann geläufigen Methoden erhalten durch Veresterung von 1-(p-Chlorbenzoyl)-5-methoxy-2-methylindol-3-essigsäure mit Tetrahydropyranyl-chloracetat, d.h. dem Chloressigsäure-tetrahydropyranylester. Der letztere ist eine neue Verbindung. Seine Herstellung kann aber nach an sich bekanntem Vorgehen erfolgen, welches als erste Stufe im nachfolgenden, der Erläuterung der Erfindung dienenden Beispiel im Detail beschrieben wird.

Zum rascheren Verständnis der Erfindung wird dem Beispiel ein Formelschema vorangestellt.

*Beispiel*

*Formelschema*

*1. Stufe*

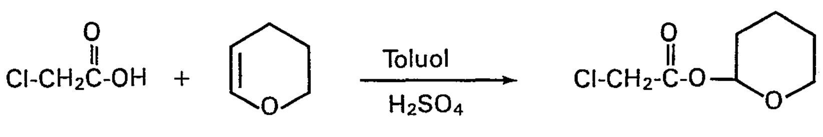

*2. Stufe*

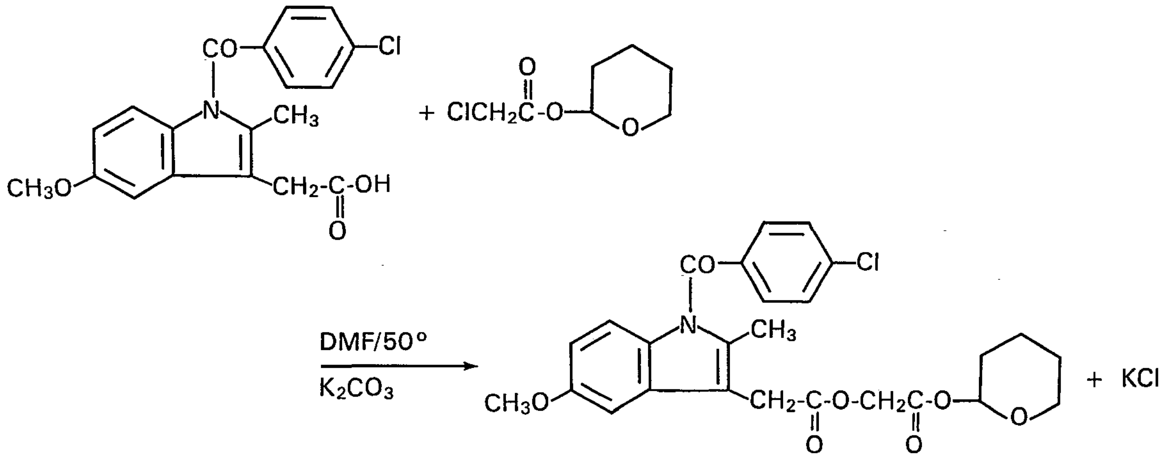


*3. Stufe*

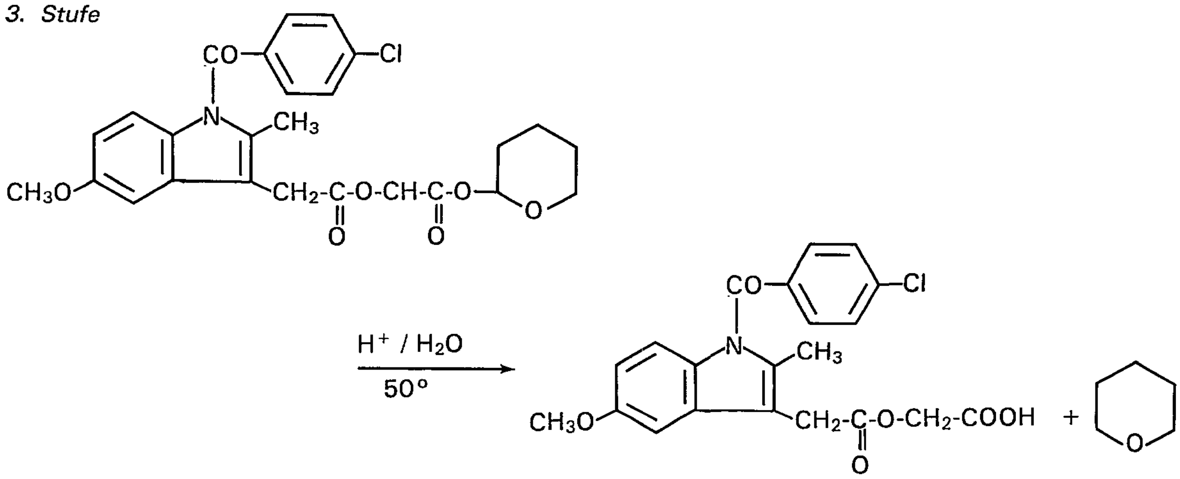


*Beschreibung der experimentellen Durchführung*

*1. Stufe: Chloressigsäure-tetrahydropyranylester:*

Zu einem Gemisch von 185 g (2,2 mol) 3,4-Dihydro-2H-pyran, 200 ml Toluol und 0,2 ml konz. Schwefelsäure wird bei 15°C eine Lösung von 142 g (1,5 mol) Chloressigsäure in 500 ml Toluol während 15 min unter Eiskühlung zugetropft. Darauf wird das Reaktionsgemisch 2 h bei Raumtemperatur gerührt, zur Stabilisierung mit 15 g wasserfreiem Kaliumcarbonat versetzt und am Rotationsverdampfer bei 45°C/Wasserstrahlvakuum zwecks Entfernung der leichtflüchtigen Anteile eingedampft. Man erhält 283 g Rückstand (Gemisch des Tetrahydropyranylesters mit Kaliumcarbonat). Ausbeute quantitativ, bezogen auf Chloressigsäure. Vor Gebrauch des Chloressigsäuresäure-tetrahydropyranylesters in der nächsten Stufe kann der Stabilisator durch Filtration abgetrennt werden.

*2. Stufe: [1-(p-Chlorphenyl)-5-methoxy-2-methylindol-3-acetoxyessigsäure]-tetrahydropyranylester (Acemetacin-tetrahydropyranylester:*

107,3 g (0,3 mol) Indometacin [1-(p-Chlorbenzoyl)-5-methoxy-2-methylindol-3-essigsäure] werden in 215 ml Dimethylformamid gelöst und bei 55°C mit 30 g (0,22 mol) wasserfreiem Kaliumcarbonat während 15 min verrührt. Darauf werden bei der gleichen Temperatur 54 g (0,3 mol) Chloressigsäure-tetrahydropyranylester der 1. Stufe während 20 min zugetropft. Nach 1 h werden weitere 18 g (0,1 mol) Tetrahydropyranylester zugegeben und 2,5 h bei 55°C gerührt. Das Reaktionsgemisch wird sodann ohne Isolierung des Acemetacin-tetrahydropyranylesters der Hydrolyse (3. Stufe) unterworfen.

*3. Stufe: Acemetacin [1-(p-Chlorbenzoyl)-5-methoxy-2-methylindol-3-acetoxyessigsäure]:*

Das Reaktionsgemisch der 2. Stufe wird mit 100 ml Essigsäure 98% und 30 ml Wasser versetzt und 2 h bei 50°C gerührt. Man kühlt auf 30°C und verdünnt mit 220 ml Wasser bis zur beginnenden Trübung, worauf nach Rühren über Nacht bei Raumtemperatur rohes Acemetacin auskristallisiert. Zur Reinigung werden die 122 g getrocknete Rohware (94%, bezogen auf das eingesetzte Indometacin) auf geeignete Art und Weise umkristallisiert, z.B. gemäss den Angaben in der DE-OS 2 943 127 durch Lösen in Aceton, Fällen des Hydrates durch Zugabe von Wasser und Trocknung bei 75°C/8 mbar, wodurch wasserfreies Acemetacin von Smp. 150-152°C erhalten wird.

**Patentansprüche**

1. Verfahren zur Herstellung von 1-(p-Chlorbenzoyl)-5-methoxy-2-methylindol-3-acetoxyessigsäure, dadurch gekennzeichnet, dass man den 1-(p-Chlorbenzoyl)-5-methoxy-2-methylindol-3-acetoxyessigsäure-tetrahydropyranylester unter Säure-Einwirkung hydrolysiert.

2. Verfahren nach Anspruch 1, dadurch gekennzeichnet, dass man zur Hydrolyse Essigsäure verwendet.

3. Verfahren nach Anspruch 2, dadurch gekennzeichnet, dass man die Hydrolyse bei einer Temperatur unter Siedepunkt des Hydrolysemediums, insbesondere im Temperaturbereich von 40 bis 60°C durchführt.

**Claims**

1. Process for preparing 1-(p-chlorobenzoyl)-5-methoxy-2-methyl-3-indolyl-acetoxy acetic acid characterized in that 1-(p-chlorobenzoyl)-5-methoxy-2-methyl-3-indolyl-acetoxy acetic acid tetrahydropyranyl ester is hydrolyzed under the influence of acid.

2. Process as claimed in claim 1, characterized in that acetic acid is used for hydrolysis.

3. Process as claimed in claim 2, characterized in that the hydrolysis is carried out at a temperature below the boiling point of the hydrolysis medium, in particular in the temperature range of 40 to 60°C.

**Revendications**

1. Procédé pour la préparation de l'acide 1-(p-chlorobenzoyl)-5-méthoxy-2-méthylindol-3-acétoxyacétique, caractérisé en ce que l'on soumet l'ester 1-(p-chlorobenzoyl)-5-méthoxy-2-méthylindol-3-acétoxyacétique du tétrahydropyranyle à une hydrolyse sous l'effet d'un acide.

2. Procédé selon la revendication 1, caractérisé en ce que l'on utilise pour effectuer l'hydrolyse l'acide acétique.

3. Procédé selon la revendication 2, caractérisé en ce que l'on conduit l'hydrolyse à une température inférieure au point d'ébullition du milieu d'hydrolyse, en particulier à une température comprise entre 40 et 60°C.